# EUROPÄISCHE PATENTANMELDUNG

(11) **EP 1 564 548 A1**
(43) Veröffentlichungstag der Anmeldung: **17.08.2005**
(21) Anmeldenummer: 04003110.6
(22) Anmeldetag: 12.02.2004
(51) Int. Cl.: G01N 22/00, G01N 22/02, G01N 22/04, G01N 33/36, D01G 31/00, D01H 13/32

(54) **In höheren Moden angeregter Mikrowellenresonator zur Messung einer dielektrischen Eigenschaft eines Produkts**

(71) Anmelder: AMS- Advanced Microwave Systems GmbH, 25336 Elmshorn (DE)
(72) Erfinder: Knöchel, Reinhard, 25336 Elmshorn (DE); Döscher, Claas, 20253 Hamburg (DE); Taute, Wolfgang, 24235 Laboe (DE)
(74) Vertreter: Patentanwälte Wenzel & Kalkoff

(57) **Zusammenfassung**

Die Anmeldung betrifft einen Mikrowellensensor zur Messung einer dielektrischen Eigenschaft, insbesondere der Dichte und/oder Feuchte eines Produkts, mit einem Mikrowellenresonator (11), wobei ein in den Resonator (11) eingeführtes Produkt (19, 31) mit einem in dem Resonator (11) erzeugten resonanten Mikrowellenfeld zur Ermittlung geeigneter Meßgrößen in Wechselwirkung steht, und zeichnet sich dadurch aus, daß in dem Resonator (11) in einer Richtung mindestens zwei Halbwellen des elektrischen Feldes ausgebildet sind, wobei die Produktzufuhrung in mindestens einen Bereich hoher Feldintensität einer der Halbwellen des elektrischen Feldes erfolgt.

## Beschreibung

Die Erfindung betrifft einen Mikrowellensensor zur Messung einer dielektrischen Eigenschaft, insbesondere der Dichte und/oder Feuchte eines Produkts, mit einem Mikrowellenresonator, wobei ein in den Resonator eingeführtes Produkt mit einem in dem Resonator erzeugten resonanten Mikrowellenfeld zur Ermittlung geeigneter Meßgrößen in Wechselwirkung steht.

Bei derartigen Sensoren ist die Feldenergie in einem bestimmten Bereich des Resonators konzentriert. Die Massebelastung des Resonators im Bereich hoher Feldenergie ist begrenzt, da eine zu hohe Massebelastung zu einer Verfälschung des Meßsignals aufgrund von Verlusten führt. Die Verwendung mehrerer Resonatoren jeweils nur für einen Teil des Produkts ist aufwendig und kann zu systematischen Fehlern aufgrund von Unterschieden der Resonatoren bzw. der entsprechenden Steuer- und Auswerteelektronik führen. Bei ausgedehnten, insbesondere bahnförmigen Produkten besteht ein weiteres Problem darin, das Produkt über eine größere Ausdehnung, beispielsweise über die gesamte Breite einer Produktbahn zu erfassen. Die Vergrößerung des Bereichs hoher Feldenergie, beispielsweise durch Vergrößerung der Wellenlänge, würde zu Bauformen mit nicht praktikablen Abmessungen führen. Die Verwendung mehrerer kleinerer Resonatoren über die Ausdehnung des Produkts ist aus den oben genannten Gründen ebenfalls nachteilig.

Aus der EP 0 889 321 A ist ein Mikrowellensensor zur Messung an einer länglichen Probe mit einem flachen, mit Dielektrikum gefüllten Resonator und einer durch den Resonator verlaufenden Durchgangsbohrung für die Probe bekannt. In dem zentral im Resonator angeordneten Meßbereich existiert ein näherungsweise homogenes Mikrowellenfeld relativ geringer Intensität.

Aus der DE 102 04 328 A ist ein Mikrowellensensor zur Bestimmung der Masse eines Faserbandes in einer Spinnereivorbereitungsmaschine bekannt, der einen Resonator mit zwei Probenvolumina für zwei Faserverbände aufweist.

Aus der WO 00/55606 A ist Mikrowellensensor mit einem resonanten Streifenleiter zur Messung der Masse eines faserigen Materials bekannt. Entlang dem Streifenleiter bildet sich eine Halbwelle des elektrischen Feldes mit Intensitätsmaxima an beiden Enden aus, an denen jeweils eine Durchführung für einen Produktstrom vorgesehen ist. Die stark inhomogenen Felder der offenen Leitungsenden sind meßtechnisch nachteilig. Weiterhin ist eine Abschirmung des Streifenleiters in Form eines metallischen Gehäuses erforderlich, um eine Verfälschung der Messung durch Strahlungsverluste zu verhindern. Das Gehäuse muß sorgfältig abgestimmt sein, um Resonanzen in der Nähe der Meßfrequenz zu vermeiden. Der Aufbau des Sensors ist daher vergleichsweise aufwendig.

Die Aufgabe der Erfindung besteht darin, einen unaufwendigen Mikrowellensensor mit verbesserter Meßgenauigkeit bereitzustellen, und vorzugsweise eine genaue Messung einer relativ großen Produktmenge und/oder eines ausgedehnten Produkts zu ermöglichen.

Die Erfindung löst diese Aufgabe insbesondere dadurch, daß in dem Resonator in einer Richtung mindestens zwei Halbwellen des elektrischen Feldes ausgebildet sind, wobei die Produktzuführung in mindestens einen Bereich hoher Feldintensität einer der Halbwellen des elektrischen Feldes erfolgt. Durch die Verwendung mindestens zweier Halbwellen des elektrischen Feldes ermöglicht die Erfindung die Reduzierung der Massebelastung des Resonators bezogen auf die gesamte im Resonator gespeicherte Energie, was zu einer Verbesserung der Meßgenauigkeit ermöglicht und bei hoher Massebelastung unter Umständen sogar einen Zusammenbruch des elektrischen Feldes verhindern kann. Erfindungsgemäß ist die Probenzuführung in einen Bereich hoher Feldenergie mindestens einer Halbwelle um ein entsprechendes Intensitätsmaximum herum angeordnet, um eine hohe Meßempfindlichkeit zu erreichen. Dies ist ein Bereich mit mindestens 50 %, vorzugsweise mindestens 70 %, weiter vorzugsweise mindestens 90 % der Intensität im Feldmaximum. Hierdurch ist die Erfindung abgegrenzt von einem höhermodigen Resonator wie aus der EP 0 889 321 A, wo die Probenzufuhr in einen zentralen Resonatorbereich mit sehr geringer Feldintensität erfolgt.

Vorzugsweise erfolgt eine Produktzuführung in mindestens zwei jeweils einer Halbwelle des elektrischen Feldes entsprechende Resonatorabschnitte. Dies ermöglicht die Verteilung des Produkts auf mehrere Halbwellen des elektrischen Feldes. Hierdurch wird die Massebelastung pro Halbwelle bzw. pro Resonatorabschnitt reduziert. Insbesondere kann durch Erhöhung der Zahl der Halbwellen die Massebelastung pro Resonatorabschnitt auf ein zweckmäßiges Maß reduziert werden. Eine relativ große Ausdehnung eines Produkts in einer Richtung kann durch Aufteilung auf mehrere Halbwellen erfaßt werden, wobei die Ausdehnung eines einzelnen Bereichs hoher Feldstärke relativ gering sein kann. Anstelle eines großen Bereichs hoher Feldstärke sind also mehrere kleinere Bereiche hoher Feldstärke vorgesehen, wodurch die Baugröße des Resonators insgesamt reduziert werden kann. Die Bereiche hoher Feldstärke werden dabei innerhalb eines Resonators erzeugt; die Verwendung mehrerer kleinerer Resonatoren ist nicht erforderlich.

Der Begriff "Halbwellen des elektrischen Feldes" bezeichnet im Falle einer bestimmten Meßfrequenz einen Bauch des cos-förmigen Feldes, d.h. ausgehend von einer Nullstelle des Feldes über ein Feldmaximum oder -minimum bis zur nächsten Nullstelle. Eine Halbwelle hat eine einer halben Wellenlänge entsprechende Ausdehnung. Die Verwendung mindestens zweier Halbwellenlängen bedeutet, daß der Hohlraumresonator in einer höheren Mode betrieben wird. Im Falle einer Mehrzahl von Meßfrequenzen kann sich der Begriff "Halbwelle" auf einen Feldbereich beziehen, der ausgehend von einer Nullstelle des Feldes über ein Feldmaximum oder -minimum bis zur nächsten Nullstelle verläuft. Alternativ kann sich der Begriff auf die Meßfrequenz mit der höchsten Intensität beziehen.

Um das Intensitätsmaximum einer Halbwelle des elektrischen Feldes existiert ein Bereich mit näherungsweise homogener Intensität, was bei Anordnung des Produkts im Intensitätsmaximum eine Verbesserung der Meßgenauigkeit ermöglicht. In vielen Fällen ist daher das Produkt in mindestens zwei Maxima des elektrischen Feldes entsprechend den mindestens zwei Halbwellen angeordnet. Wenn für sämtliche Teile des Produkts ein gleichgewichteter Mittelwert der Meßgröße erhalten werden soll, sind die Produktteile in jedem Resonatorabschnitt zweckmäßigerweise im wesentlichen in dem jeweiligen Maximum des elektrischen Feldes angeordnet. Durch gezielte Abweichung der Anordnung einzelner Produktteile vom Maximum in den jeweiligen Resonatorabschnitten kann eine beliebig gewünschte Gewichtung der einzelnen Produktteile eingestellt werden.

Der Resonator kann unterschiedliche Bauformen aufweisen. Vorzugsweise handelt es sich um einen geschirmten Resonator bzw. Hohlraumresonator, d.h. ein von metallischen Wänden begrenzter Hohlraum, der abgesehen von Öffnungen für die Probenzuführung im wesentlichen geschlossen ist. Bei einem Hohlraumresonator wird intern eine Hohlraumresonanz angeregt. Diese Bauweise ist besonders unaufwendig beispielsweise im Vergleich zu dem Streifenleiter der WO 00/55606 A. Besonders einfach und daher bevorzugt ist ein Rechteckresonator. Es kann sich auch um einen insbesondere über Chokes geöffneten Resonator handeln. Die Erfindung betrifft jedoch nur solche Resonatoren, bei denen das Produkt zur Messung in den Resonatorraum eingeführt oder durch diesen hindurchgeführt wird. Der Gegenstand der Anmeldung ist daher abgegrenzt gegenüber solchen (Streufeld-)Resonatoren, bei denen die Messung mittels eines externen elektrischen Streufeldes erfolgt, so daß ein vollkommen andersartiger Feldverlauf vorliegt.

Besonders nützlich ist die Erfindung für die Messung an einem durch den Resonator geführten kontinuierlichen Produktstrom, da hier die Produktmasse bzw. die Produktausdehnung vorgegeben ist und nicht auf den Resonator abgestimmt werden kann. Umfaßt sind insbesondere strang- und bandförmige Produkte, beispielsweise Faserprodukte, Tabak, Papier und dergleichen. Umfaßt ist aber auch die Messung an einzelnen Produktportionen. Bei einem strangförmigen Produkt sind die mindestens zwei Resonatorabschnitte zweckmäßigerweise zur Durchführung jeweils mindestens eines Produktstranges eingerichtet. Es kann vorteilhaft sein, wenn durch jeden Resonatorabschnitt zwei oder sogar mehr Produktstränge geführt werden, da beim Reißen eines Faserstranges dieser von dem anderen Strang mitgeführt wird. Allerdings werden vorzugsweise nicht mehr als zwei Produktstränge durch jeden Resonatorabschnitt geführt, um die entsprechende Massebelastung möglichst gering zu halten.

Zahl und Anordnung der Resonatorabschnitte können frei an die jeweiligen Anforderungen angepaßt werden. Es kann sich um eine eindimensionale, insbesondere lineare Anordnung beispielsweise quer über die Breite einer Produktbahn handeln. Auch eine ringförmige Anordnung ist umfaßt, wobei die mindestens zwei Halbwellen in Umfangsrichtung angeordnet sind. Es kann sich auch um eine zweidimensionale Anordnung handeln, bei der in zwei Richtungen jeweils mindestens zwei Halbwellen des elektrischen Feldes im Resonator vorgesehen sind. Selbst eine dreidimensionale Anordnung ist möglich. Die Zahl der Resonatorabschnitte beträgt vorzugsweise mindestens drei, vorzugsweise mindestens vier. Es ist möglich, nicht benötigte Resonatorabschnitte leer laufen zu lassen. Beispielsweise kann ein Vierstrangprodukt mit einem Resonator mit vier Resonatorabschnitten gemessen werden, indem jeweils zwei Stränge durch zwei Resonatorabschnitte geführt werden und die beiden übrigen Resonatorabschnitte leer laufen. Es kann daher dieselbe Resonatorbauform (beispielsweise Resonator mit vier Resonatorabschnitten) für unterschiedliche Anwendungen (im genannten Beispiel ein bis acht Stränge) verwendet werden. Gegebenenfalls müssen nicht sämtliche Resonatorabschnitte eine Probenzuführung aufweisen.

Der Resonator, gegebenenfalls der Hohlraumresonator, kann mit einem festen Dielektrikum mit einer Dielektrizitätszahl größer als eins, vorzugsweise mindestens zwei, weiter vorzugsweise mindestens fünf gefüllt sein. Übliche Materialien, insbesondere Keramiken, sind dem Fachmann bekannt. Die Verwendung einer dielektrischen Füllung erlaubt die Speicherung von Feldenergie auf vergleichsweise kleineren Raum und ermöglicht daher kleinere Bauformen. Vorzugsweise ist im wesentlichen der gesamte Resonator bis auf den Raum zur Aufnahme der Probe mit Dielektrikum gefüllt. "Im wesentlichen" bedeutet abgesehen von Einrichtungen zum Einkoppeln der Mikrowellen, zum Durchführen der Probe und dergleichen. Der Resonator kann jedoch auch im wesentlichen mit Luft gefüllt sein, was einen besonders einfachen und unaufwendigen Aufbau darstellt.

Weitere vorteilhafte Merkmale und Ausführungsformen der Erfindung gehen aus den Unteransprüchen und der folgenden Beschreibung der Erfindung unter Bezugnahme auf die beigefügten Zeichnungen hervor. Dabei zeigen:
- Fig. 1:: eine Draufsicht auf einen Sensor gemäß einer ersten Bauform;
- Fig. 2:: eine Ansicht des Sensors aus Fig. 1 von vorne;
- Fig. 3:: eine Ansicht eines Sensors gemäß einer zweiten Bauform von vorne;
- Fig. 4:: eine Ansicht eines Sensors gemäß einer dritten Bauform von vorne;
- Fig. 5:: eine Draufsicht auf einen Sensor gemäß einer vierten Bauform;
- Fig. 6:: eine Ansicht des Sensors aus Fig. 5 von vorne;
- Fig. 7:: eine Draufsicht eines Sensors gemäß einer fünften Bauform;
- Fig. 8:: eine schematische Skizze einer Meßanordnung mit einem Sensor; und
- Fig. 9:: eine Draufsicht eines Sensors gemäß einer sechsten Bauform.

Ein Mikrowellensensor 10 weist einen Hohlraumresonator 11 auf, in dem ein stehendes Mikrowellenfeld erzeugt wird. Die Ein- und Auskopplung des Mikrowellenfeldes erfolgt mittels Koppeleinrichtungen 12. Mikrowellen werden mittels eines von einem Rechner 13 gesteuerten Generators 14 erzeugt und mittels einer Leitung zu der Koppeleinrichtung 12a geleitet. Über die Koppeleinrichtung 12b wird ein Mikrowellensignal aus dem Resonator 11 ausgekoppelt und mittels einer Leitung einem Analysator 15 zugeführt, dessen Ausgangssignal vom Rechner 13 verarbeitet werden kann. Generator 14, Analysator 15 und Rechner 13 sind zweckmäßigerweise in einer Meßeinheit 16 zusammengefaßt.

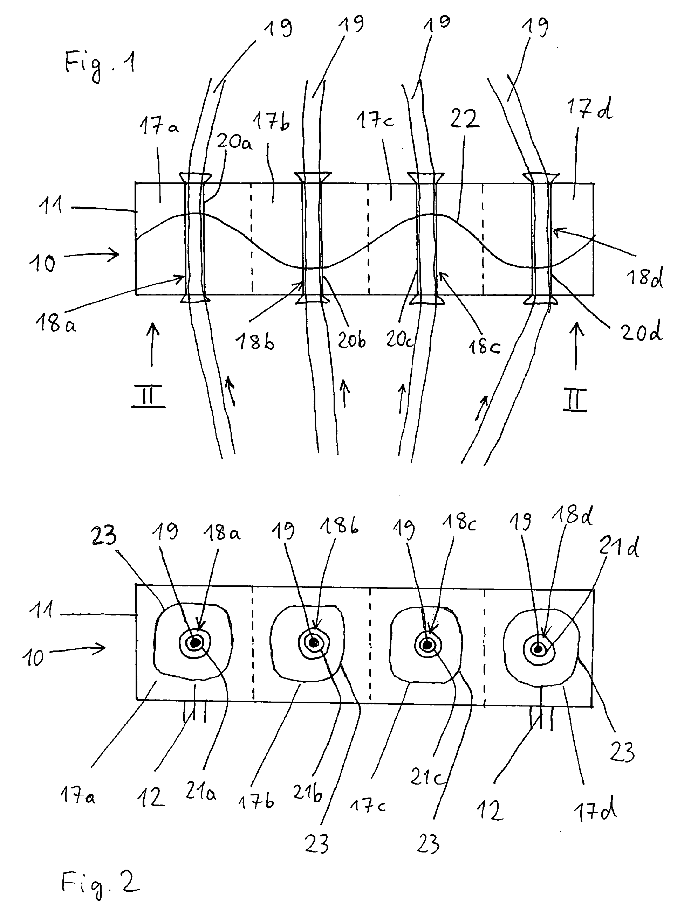

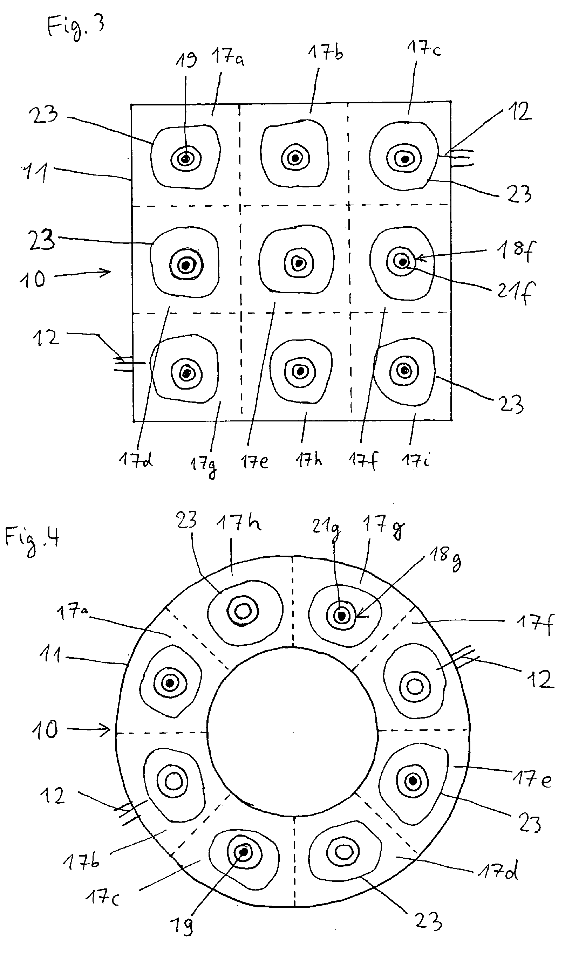

Der Resonator 11 weist eine Mehrzahl von Resonatorabschnitten 17a, 17b, 17c, ... auf. Die Trennung in verschiedene Resonatorabschnitte ist in den Figuren mit gestrichelten Linien angedeutet. Die Fig. 1, 2, 5, 6, 7, 9 betreffen jeweils längliche Rechteck-Hohlraumresonatoren mit beispielsweise vier bzw. zwei Resonatorabschnitten. Länglich bedeutet, daß die Ausdehnung entlang einer Längsachse um mindestens einen Faktor 2, vorzugsweise mindestens einen Faktor 3, weiter vorzugsweise mindestens eine Faktor 5 länger ist als die Ausdehnung entlang der beiden zur Längsachse senkrechten Richtungen. Die Resonatorabschnitte 17 sind in Serie entlang der Längsachse des Resonators 11 angeordnet. Fig. 3 zeigt einen Resonator, bei dem die Resonatorabschnitte in Spalten und Zeilen, d.h. in Form einer Matrix angeordnet sind. Die Zahl der Spalten, Reihen sowie allgemeiner die Anordnung der Resonatorabschnitte kann nach Belieben den jeweiligen Erfordernissen angepaßt werden. Fig. 4 betrifft einen ringförmigen Resonator 11, bei dem die Resonatorabschnitte 17 in Serie entlang der Ringachse des Resonators 11 angeordnet sind. In diesem Beispiel laufen vier Resonatorabschnitte 17b, 17d, 17f, 17h leer. Im allgemeinen kann eine beliebige Anzahl von Resonatorabschnitten 17 leer laufen.

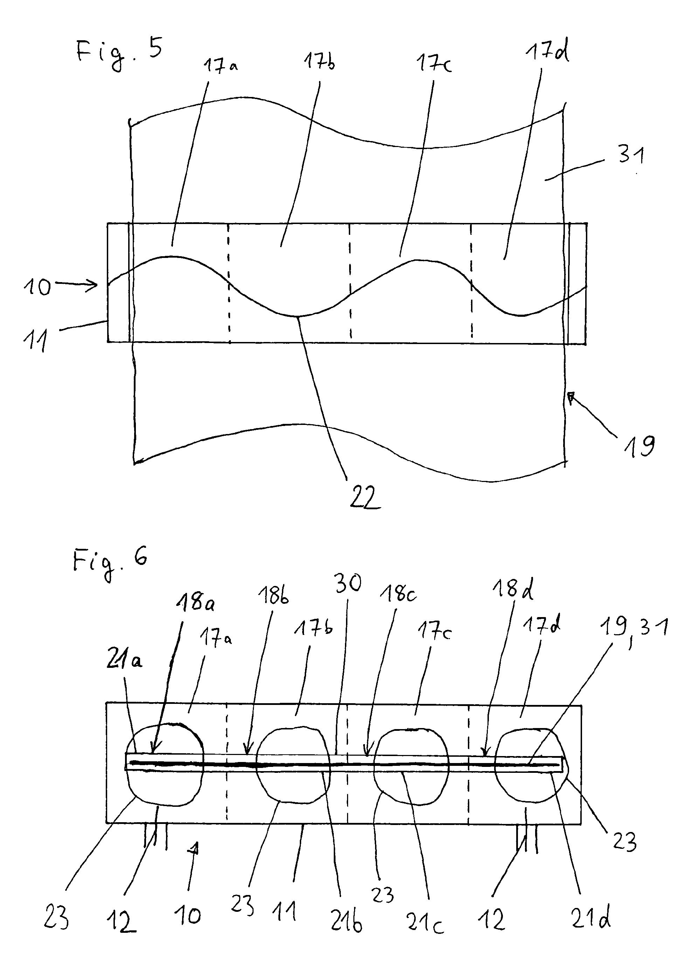

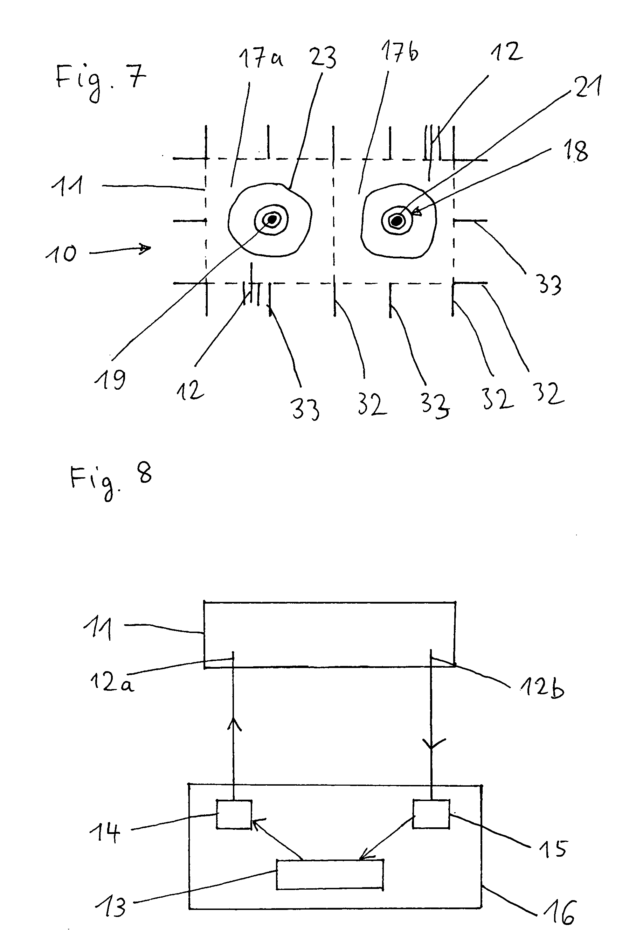

Für die Probenzuführung weist jeder Resonatorabschnitt Öffnungen 21a, 21b, 21c, ..., auf, die insbesondere in den Resonator begrenzenden Wänden vorgesehen sein können. Bei Messungen an einem Produktstrom, insbesondere an einem strangförmigen Produktstrom (Fig. 1 bis 4 und 7) oder einem bandförmigen Produktstrom (Fig. 5, 6), sind zweckmäßigerweise jeweils separate Öffnungen 21 für den Eintritt und Austritt der Probe 19 in den bzw. aus dem Resonator 11 vorgesehen. Vorzugsweise entspricht die Größe der Öffnungen 21 etwa dem Querschnitt des zu messenden Produkts 19. Vorzugsweise ist die Form der Öffnungen 21 dem Produktquerschnitt angepaßt. Beispielsweise im Falle eines strangförmigen Produkts sind daher die Öffnungen 21 vorzugsweise kreisrund. Vorzugsweise ist der Resonator bis auf die Produktzuführungsöffnungen 21 im wesentlichen geschlossen. Geschlossen bedeutet für die verwendeten Mikrowellen undurchlässig. Die oben genannten Merkmale tragen zur Reduzierung der nachteiligen Abstrahlung von Mikrowellenenergie aus dem Resonator bei.

Zur Durchführung des Produkts 19 durch den Resonator 11 bzw. die Resonatorabschnitte 17 können Probenfiihrungseinrichtungen 18a, 18b, 18c, ... vorgesehen sein, beispielsweise Röhrchen 20a, 20b, 20c, ..., die vorzugsweise aus verlustarmem Dielektrikum mit geringer Temperaturabhängigkeit, beispielsweise Quarzglas bestehen können.

Die vom Generator 14 erzeugte Mikrowellenfrequenz ist so abgestimmt, daß sich in dem Resonator 11 eine stehende Welle mit mindestens zwei bauchigen Halbwellen mit jeweils einem lokalen Intensitätsmaximum ausbildet. In den Fig. 1, 5, 9 sind beispielhaft elektrische Feldlinien 22 für den Fall einer Mikrowelle mit bestimmter Frequenz eingezeichnet. In diesem Fall breitet sich pro Resonatorabschnitt 17 eine cos-förmige Halbwelle des elektrischen Feldes aus. Beliebige andere Feldverteilungen mit mindestens zwei Halbwellen, auch mit mehr als einer Frequenz, im allgemeinen mit beliebiger Frequenzverteilung, und/oder mehreren Maxima pro Resonatorabschnitt sind ebenfalls möglich. In den Fig. 2 bis 4, 6, 7 sind Linien 23 konstanter Feldintensität eingezeichnet. Innerhalb der Linien 23 liegt jeweils ein Bereich hoher Feldintensität mit einem lokalen Intensitätsmaximum, das etwa im Zentrum des jeweiligen Resonatorabschnitts 17 liegt. Dies ist aber im allgemeinen nicht zwingend erforderlich. Außerhalb der Linien 23 ist die Feldintensität geringer als innerhalb.

Durch die Verteilung des gesamten Produktstroms 19 auf eine Mehrzahl von Resonatorabschnitten 17 ist die Massebelastung pro Resonatorabschnitt 17 entsprechend reduziert, im Beispiel der Fig. 1 beispielsweise um den Faktor 4, im Beispiel der Fig. 3 beispielsweise um den Faktor 9. Die einzelnen Teile der Probe 19 werden jeweils durch einen eng um das jeweilige Intensitätsmaximum angeordneten Bereich des jeweiligen Resonatorabschnitts 17 geführt. Wie beispielsweise in Fig. 1 ersichtlich, ist in diesem Bereich die Feldstärke etwa konstant. Positions- und Orientierungsveränderungen der Probe sowie räumlichen Inhomogenitäten innerhalb des Produktstroms wirken sich daher nicht oder höchstens wenig auf das Meßsignal aus, da sämtliche Teile des Produktstroms mit derselben Gewichtung - aufgrund der etwa konstanten Feldstärke - in das Meßsignal eingehen, wie in den Beispielen der Fig. 1 bis 4, 7, 9 ersichtlich. Eine Öffnung 21 kann gezielt vom jeweiligen Intensitätsmaximum abweichend angeordnet sein, um individuelle Gewichtungen einzelner Produktteile zu ermöglichen.

Die Ausführungsform gemäß Fig. 5, 6 betrifft die Messung an einem flächigen Produkt 31, insbesondere einem platten- oder bahnförmigen Produkt, beispielsweise Papier. Die Öffnung 21 ist schlitzförmig, wobei der Schlitz 30 aus Schlitzabschnitten 21a, 21b, 21c, 21d zusammengesetzt ist. Die Länge des Schlitzes 30 ist an die Breite der Papierbahn 31 angepaßt. Zur Vermessung einer doppelt so langen Papierbahn 31 kann dieselbe Meßeinheit 16 bei derselben Mikrowellenfrequenz verwendet werden, indem einfach eine lineare Anordnung von acht statt vier Resonatorabschnitten 17 gewählt wird, wodurch sich die Länge des Resonators 11 etwa verdoppeln würde. Auf diese Weise muß die Baugröße des Resonators 11 nur entlang der Längsachse, d.h. nur ein einer Dimension, verändert werden, während die Baugröße des Resonators 11 ansonsten unverändert bleiben kann.

Generell ist es nicht erforderlich, daß der Schlitz 30, wie im Beispiel der Fig. 5, 6, geschlossen ist. Der Resonator 11 kann aus zwei getrennten Resonatorhälften bestehen, zwischen denen ein Probenzuführungsschlitz gebildet ist. Vorzugsweise sind die Mikrowellen-Koppeleinrichtungen 12 jeweils in verschiedenen Resonatorhälften angeordnet. Es ist auch möglich, strangförmige Produkte mit einem geschlitzten Resonator zu messen.

Die in Fig. 7 gezeigte Ausführungsform verdeutlicht, daß die Wände des Resonators 11 nicht vollkommen geschlossen, sondern lediglich für Mikrowellen im wesentlichen undurchlässig sein sollen. Im Fall der Fig. 7 sind Trenneinrichtungen 32, 33, beispielsweise Trennbleche vorgesehen, die vorzugsweise periodisch in einem Abstand voneinander angeordnet sind, der geringer ist als die der Ausbreitungs-Grenzfrequenz entsprechende Wellenlänge des Mikrowellenfeldes. Das Feld kann zwischen den Trennblechen 32 etwas herauslappen, ist jedoch insgesamt in wesentlichen in dem Innenraum des Resonators 11 konzentriert.

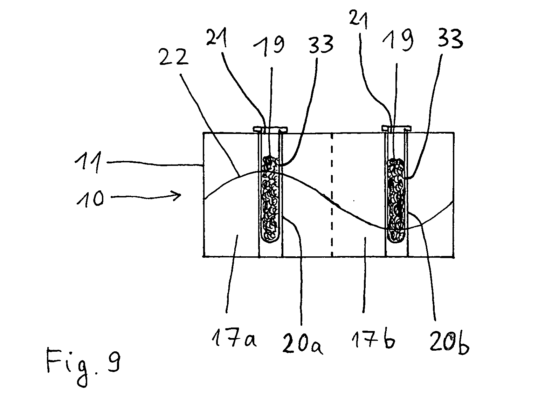

Die in Fig. 9 gezeigte Ausführungsform betrifft einen Resonator 11 zur Messung an einem portionierten Produkt 19. Die einzelnen Portionen können sich in einem Probenbehälter 33, beispielsweise einem Quarzglasröhrchen, befinden. Der Resonator 11 weist pro Resonatorabschnitt 17a, 17b lediglich eine Öffnung 21 in den Resonatorwänden zum Einführen des Produkts 19 auf. Dies kann automatisiert beispielsweise mittels eines Roboters erfolgen. Führungseinrichtungen 20 sind bevorzugt, aber nicht zwingend vorgesehen. Auch in diesem Beispiel kann bei relativ geringer Massenbelastung pro Resonatorabschnitt 17 mit nur einem Resonator 11 eine relativ große Probenmasse gemessen werden.

## Patentansprüche

1. Mikrowellensensor zur Messung einer dielektrischen Eigenschaft, insbesondere der Dichte und/oder Feuchte eines Produkts, mit einem Mikrowellenresonator (11), wobei ein in den Resonator (11) eingeführtes Produkt (19, 31) mit einem in dem Resonator (11) erzeugten resonanten Mikrowellenfeld zur Ermittlung geeigneter Meßgrößen in Wechselwirkung steht, **dadurch gekennzeichnet, daß** in dem Resonator (11) in einer Richtung mindestens zwei Halbwellen des elektrischen Feldes ausgebildet sind, wobei die Produktzuführung in mindestens einen Bereich hoher Feldintensität einer der Halbwellen des elektrischen Feldes erfolgt.

2. Mikrowellensensor nach Anspruch 1, **dadurch gekennzeichnet, daß** eine Produktzuführung in mindestens zwei jeweils einer Halbwelle des elektrischen Feldes entsprechende Resonatorabschnitte (17a, 17b, 17c, ...) erfolgt.

3. Mikrowellensensor nach Anspruch 1, **dadurch gekennzeichnet, daß** der Resonator (11) ein Hohlraumresonator ist.

4. Mikrowellensensor nach Anspruch 1 oder 2, **dadurch gekennzeichnet, daß** er zur Messung an einem durch den Resonator (11) geführten Produktstrom eingerichtet ist.

5. Mikrowellensensor nach einem der Ansprüche 1 bis 3, **dadurch gekennzeichnet, daß** Resonatoröffnungen (21 a, 21b, 21c, ...) für die Probenzuführung im wesentlichen dem Querschnitt des zuzuführenden Produkts (19, 31) entsprechen.

6. Mikrowellensensor nach einem der Ansprüche 1 bis 4, **dadurch gekennzeichnet, daß** die Zahl der Halbwellenlängen des elektrischen Feldes mindestens drei, vorzugsweise mindestens vier beträgt.

7. Mikrowellensensor nach einem der Ansprüche 1 bis 5, **dadurch gekennzeichnet, daß** die Produktzuführung jeweils durch einen Bereich hoher Feldintensität der Halbwellen des elektrischen Feldes verläuft.

8. Mikrowellensensor nach einem der Ansprüche 1 bis 6, **dadurch gekennzeichnet, daß** die Produktzuführung jeweils durch das Intensitätsmaximum der Halbwellen des elektrischen Feldes verläuft.

9. Mikrowellensensor nach einem der Ansprüche 1 bis 7, **dadurch gekennzeichnet, daß** die Position der Produktzuführung relativ zu einem Intensitätsmaximum des elektrischen Feldes verstellbar ist.

10. Mikrowellensensor nach einem der Ansprüche 1 bis 8, **dadurch gekennzeichnet, daß** die jeweils einer Halbwelle des elektrischen Feldes entsprechenden Resonatorabschnitte (17a, 17b, 17c, ...) linear angeordnet sind.

11. Mikrowellensensor nach einem der Ansprüche 1 bis 9, **dadurch gekennzeichnet, daß** der Resonator (11) mit einem Dielektrikum gefüllt ist.

12. Mikrowellensensor nach einem der Ansprüche 1 bis 10, **dadurch gekennzeichnet, daß** für jede Halbwelle des elektrischen Feldes eine separate Probenzuführung vorgesehen ist.

13. Mikrowellensensor nach einem der Ansprüche 1 bis 11, **dadurch gekennzeichnet, daß** jeder einer Halbwelle des elektrischen Feldes entsprechende Resonatorabschnitt (17a, 17b, 17c, ...) zur Zuführung mindestens zweier Produktstränge oder -bänder vorgesehen ist.

14. Mikrowellensensor nach einem der Ansprüche 1 bis 10, **dadurch gekennzeichnet, daß** er einen sich über mehrere Halbwellen erstreckenden Schlitz (30) zur Probendurchführung aufweist.

15. Mikrowellensensor nach einem der Ansprüche 1 bis 10, **dadurch gekennzeichnet, daß** er zur Messung an einem flächigen Produkt (31) eingerichtet ist.

16. Mikrowellensensor nach Anspruch 14, **dadurch gekennzeichnet, daß** die Zahl der Halbwellen des elektrischen Feldes an die Breite des flächigen Produkts (31) angepaßt ist.

17. Mikrowellensensor nach einem der Ansprüche 1 bis 10, **dadurch gekennzeichnet, daß** der Resonator (11) aus zwei durch einen Probenzuführungsschlitz getrennten Resonatorhälften besteht.
